# EUROPEAN PATENT APPLICATION

(11) **EP 4 481 032 A1**
(43) Date of publication of application: **25.12.2024**
(21) Application number: 22927283.6
(22) Date of filing: 31.10.2022
(51) Int. Cl.: C12M 3/00

(54) **MICROFLUIDIC DEVICE AND METHOD FOR USING MICROFLUIDIC DEVICE**

(30) Priority: 16.02.2022 JP 2022022345
(71) Applicant: Ushio Denki Kabushiki Kaisha, Tokyo 100-8150 (JP)
(72) Inventor: TAKAHASHI, Seiichiro, Tokyo 100-8150 (JP)
(74) Representative: Maiwald GmbH
(86) International application number: PCT/JP2022/040586
(87) International publication number: WO 2023/157389

(57) **Abstract**

Provide are a microfluidic device capable of constructing a gel surface that enables cell seeding and has excellent observability and a method for using the microfluidic device. A microfluidic device includes: a plate-shaped main body having a first principal surface and a second principal surface facing each other in a first direction; a first flow path formed inside the main body and extending along a plane between the first principal surface and the second principal surface; and a plurality of first flow path ports extending in the first direction and each having one end open to an end of the first flow path and another end open to the second principal surface, wherein the first flow path has an enlarged part so that a flow path width increases from a first principal surface side toward a second principal surface side when the first flow path is viewed in its extension direction.

## Description

### TECHNICAL FIELD

The present invention relates to a microfluidic device and a method for using the microfluidic device.

### BACKGROUND ART

3D gel culture (three-dimensional gel culture) allows cells to three-dimensionally grow, which makes it possible to carry out a study using a structure closer to in vivo conditions as compared to conventional two-dimensional cell culture. Therefore, study results unique to 3D gel culture are expected, such as tissue formation, such as organoid formation, that cannot be performed by conventional two-dimensional culture, application to regenerative medicine, analysis of pharma-cokinetics in a three-dimensional environment close to in vivo conditions, and a study of cell-to-cell interactions.

As a device for 3D gel culture, such a device using a cell culture insert as disclosed in Patent Document 1 mentioned below is generally used. On the other hand, a device using micro flow paths (MPS) (hereinafter, also referred to as a microfluidic device) disclosed in Patent Document 2 mentioned below has been developed as a new technology.

### PRIOR ART DOCUMENTS

### PATENT DOCUMENTS

Patent Document 1: JP-A-2020-202754
Patent Document 2: JP-T-2018-522586

### SUMMARY OF THE INVENTION

### PROBLEMS TO BE SOLVED BY THE INVENTION

Such a conventional device having a cell culture insert has an advantage that a flat gel surface that enables cell seeding and has excellent observability can be constructed in the device, but has a problem that advantages of a microfluidic device cannot be adopted, such as that the size, shape, and oxygen concentration of a cell culture environment can be made close to those in vivo. On the other hand, a microfluidic device has the above-described unique advantage, but has a problem that a flat gel surface excellent in observability cannot be constructed in the device.

In light of the above problems, it is an object of the present invention to provide a microfluidic device capable of constructing a gel surface that enables cell seeding and has excellent observability and a method for using the microfluidic device.

### MEANS FOR SOLVING THE PROBLEMS

The present invention is directed to a microfluidic device including: a plate-shaped main body having a first principal surface and a second principal surface facing each other in a first direction; a first flow path formed inside the main body and extending along a plane between the first principal surface and the second principal surface; and a plurality of first flow path ports extending in the first direction and each having one end open to an end of the first flow path and another end open to the second principal surface, in which
the first flow path has an enlarged part so that a flow path width increases from a first principal surface side toward a second principal surface side when the first flow path is viewed in its extension direction.

Such a microfluidic device enables cell seeding on the top surface of a gel because a space is maintained above the gel formed in the first flow path, and is excellent in observability because a flat gel surface can be formed.

The present invention is also directed to a method for using the above-described microfluidic device, the method including:
a first step in which the microfluidic device is disposed so that the first principal surface is located on a lower side and the second principal surface is located on an upper side;
a second step in which a sol is injected into a region below the enlarged part in the first flow path, and
a third step in which cells are seeded on a top surface of a gel obtained by solidifying the sol.

Such a method for using the microfluidic device enables cell seeding on the top surface of a gel because a space is maintained above the gel formed in the first flow path, and is excellent in observability because a flat gel surface can be formed.

### BRIEF DESCRIPTION OF THE DRAWINGS

Fig. 1 is a perspective view of a microfluidic device according to a first embodiment.
Fig. 2 is a sectional view of the microfluidic device taken along a line II-II shown in Fig. 1.
Fig. 3 is a sectional view of the microfluidic device taken along a line III-III shown in Fig. 1.
Fig. 4A is a sectional view for illustrating a method for using the microfluidic device shown in Fig. 1.
Fig. 4B is a sectional view for illustrating a method for using the microfluidic device shown in Fig. 1.
Fig. 4C is a sectional view for illustrating a method for using the microfluidic device shown in Fig. 1.
Fig. 4D is a sectional view for illustrating a method for using the microfluidic device shown in Fig. 1.
Fig. 5 is a sectional view for illustrating a method for using the microfluidic device shown in Fig. 1.
Fig. 6 is a sectional view of a microfluidic device of a comparative example.
Fig. 7 is a perspective view of a microfluidic device according to a second embodiment.
Fig. 8 is a sectional view of the microfluidic device taken along a line VIII-VIII shown in Fig. 7.
Fig. 9 is a sectional view of the microfluidic device taken along a line IX-IX shown in Fig. 7.
Fig. 10 is a sectional view of a microfluidic device according to another embodiment.
Fig. 11 is a sectional view of a microfluidic device according to another embodiment.
Fig. 12 is a sectional view of a microfluidic device according to another embodiment.
Fig. 13 is a sectional view of a microfluidic device according to another embodiment.
Fig. 14 is a sectional view of a microfluidic device according to another embodiment.
Fig. 15 is a sectional view of a microfluidic device according to another embodiment.
Fig. 16 is a sectional view of a microfluidic device according to another embodiment.
Fig. 17 is a sectional view of a microfluidic device according to another embodiment.
Fig. 18 is a sectional view of a microfluidic device according to another embodiment.
Fig. 19 is a plan view of a microfluidic device according to another embodiment.
Fig. 20 is a sectional view of the microfluidic device taken along a line XX-XX shown in Fig. 19.
Fig. 21A is a sectional view for illustrating a method for using the microfluidic device shown in Fig. 19.
Fig. 21B is a sectional view for illustrating a method for using the microfluidic device shown in Fig. 19.
Fig. 21C is a sectional view for illustrating a method for using the microfluidic device shown in Fig. 19.
Fig. 21D is a sectional view for illustrating a method for using the microfluidic device shown in Fig. 19.

### MODES FOR CARRYING OUT THE INVENTION

A microfluidic device and a method for using the microfluidic device according to the present invention will be described with reference to the drawings. It should be noted that the drawings disclosed herein merely show schematic illustrations. Namely, the dimensional ratios on the drawings do not necessarily reflect the actual dimensional ratios, and the dimensional ratios are not necessarily the same between the drawings.

### <First embodiment>

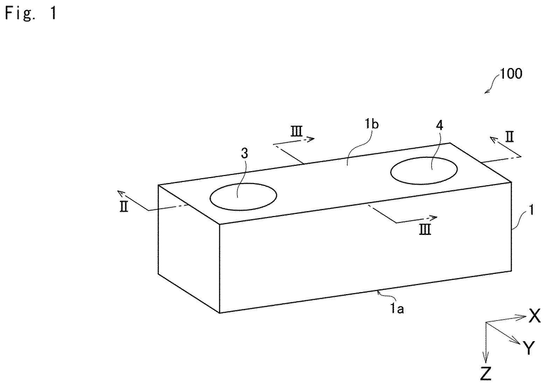

Fig. 1 is a perspective view of a microfluidic device 100 according to a first embodiment. The microfluidic device 100 includes a main body 1 having a first principal surface 1a and a second principal surface 1b. The first principal surface 1a and the second principal surface 1b are disposed so as to face each other.

In the following description, an XYZ coordinate system is appropriately referenced in which a plane parallel to the first principal surface 1a and the second principal surface 1b is defined as an XY plane and a direction orthogonal to the XY plane is defined as a Z direction.

When it is necessary to make a distinction between positive and negative to express a direction herein, the direction is described with a positive or negative sign, such as "+X direction" or "-X direction". When it is not necessary to make a distinction between positive and negative to express a direction, the direction is simply described as "X direction". Namely, when the direction is simply described as "X direction" herein, both "+X direction" and "-X direction" are included. The same applies to a Y direction and a Z direction.

The main body 1 is formed in a plate shape so as to have the first principal surface 1a and the second principal surface 1b facing each other in the Z direction. The Z direction in the present embodiment corresponds to a "first direction". It should be noted that the microfluidic device 100 is usually used so that the Z direction corresponds to a vertical direction, and the -Z direction corresponds to an upward direction.

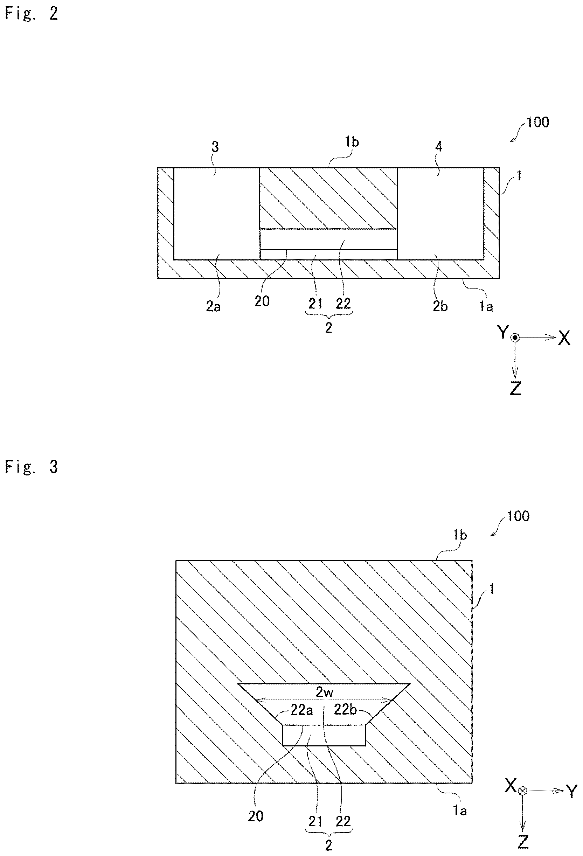

As shown in Fig. 2, the microfluidic device 100 includes a first flow path 2 formed inside the main body 1. The first flow path 2 extends along the XY plane between the first principal surface 1a and the second principal surface 1b, and specifically extends in the X direction. Both ends 2a and 2b of the first flow path 2 are located inside the microfluidic device 100.

The microfluidic device 100 includes first flow path ports 3 and 4 extending in the Z direction. The first flow path port 3 is connected to the one end 2a of the first flow path 2, and the first flow path port 4 is connected to the other end 2b of the first flow path 2. The first flow path port 3 has one end open to the one end 2a of the first flow path 2 and another end open to the second principal surface 1b. The first flow path port 4 has one end open to the other end 2b of the first flow path 2 and another end open to the second principal surface 1b. The first flow path ports 3 and 4 have at least one purpose selected from the purpose of injecting a liquid into the microfluidic device 100 and the purpose of discharging a liquid from the microfluidic device 100. For example, the first flow path port 3 may be used as a liquid inlet and the first flow path port 4 may be used as a liquid outlet.

As shown in Fig. 3, the first flow path 2 of the first embodiment includes a first space 21 and a second space 22 adjacent to the first space 21 on the second principal surface 1b side in the Z direction (-Z direction side). The first space 21 has a rectangular cross-section in a YZ plane. The second space 22 has a trapezoidal cross-section in the YZ plane. The second space 22 has side walls 22a and 22b, and the side walls 22a and 22b are inclined with respect to the Z direction so as to separate from each other as extending toward the second principal surface 1b. Therefore, the side walls 22a and 22b of the second space 22 allow a flow path width 2w of the first flow path 2 in the Y direction to increase from the first principal surface 1a side toward the second principal surface 1b side. That is, the side walls 22a and 22b of the second space 22 in the first embodiment correspond to "enlarged parts" in the present invention.

A virtual surface 20 formed by connecting the first principal surface 1a-side end of the side wall 22a and the first principal surface 1a-side end of the side wall 22b, specifically an interface 20 between the first space 21 and the second space 22 is parallel to the first principal surface 1a.

As described above, the microfluidic device 100 according to the first embodiment includes a plate-shaped main body 1 having a first principal surface 1a and a second principal surface 1b facing each other in the Z direction, a first flow path 2 formed inside the main body 1 and extending along the XY plane between the first principal surface 1a and the second principal surface 1b, and first flow path ports 3 and 4 extending in the Z direction and each having one end open to an end 2a or 2b of the first flow path 2 and another end open to the second principal surface 1b. Further, the first flow path 2 has side walls 22a and 22b (enlarged parts) so that a flow path width 2w increases from the first principal surface 1a side toward the second principal surface 1b side when the first flow path 2 is viewed in its extension direction (X direction). The function and effect of the microfluidic device 100 will be described later in the description of a method for using the microfluidic device 100.

Further, as described with reference to the first embodiment, the first flow path 2 includes a first space 21 and a second space 22 that is adjacent to the first space 21 on the second principal surface 1b side in the Z direction and that has a larger flow path width 2w than the first space 21 by the side walls 22a and 22b (enlarged parts).

Further, as described with reference to the first embodiment, the side walls 22a and 22b (enlarged parts) provide surfaces inclined with respect to the first principal surface 1a.

Further, as described with reference to the first embodiment, the first flow path 2 has side walls 22a and 22b (enlarged parts) on its both sides in the Y direction respectively, and a virtual surface 20 formed by connecting together the first principal surface 1a-side end of the side wall 22a and the first principal surface 1a-side end of the side wall 22b is parallel to the first principal surface 1a.

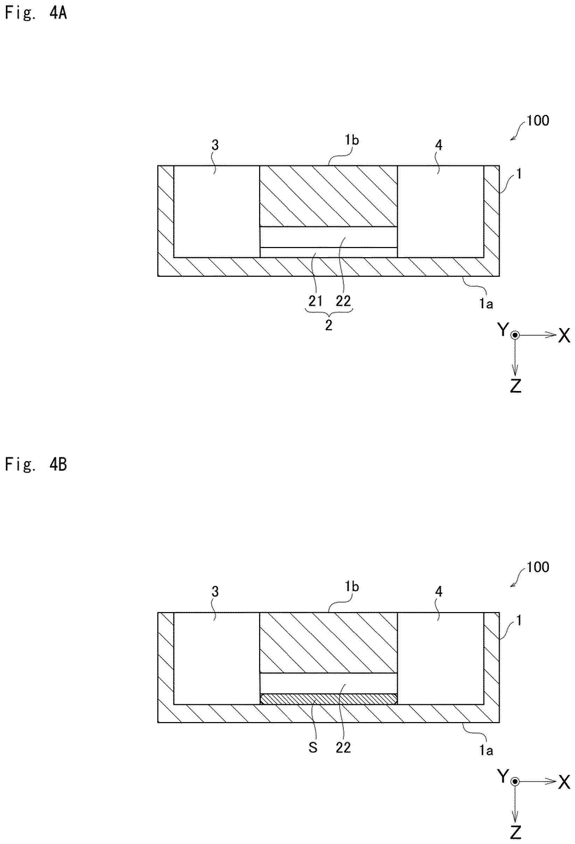

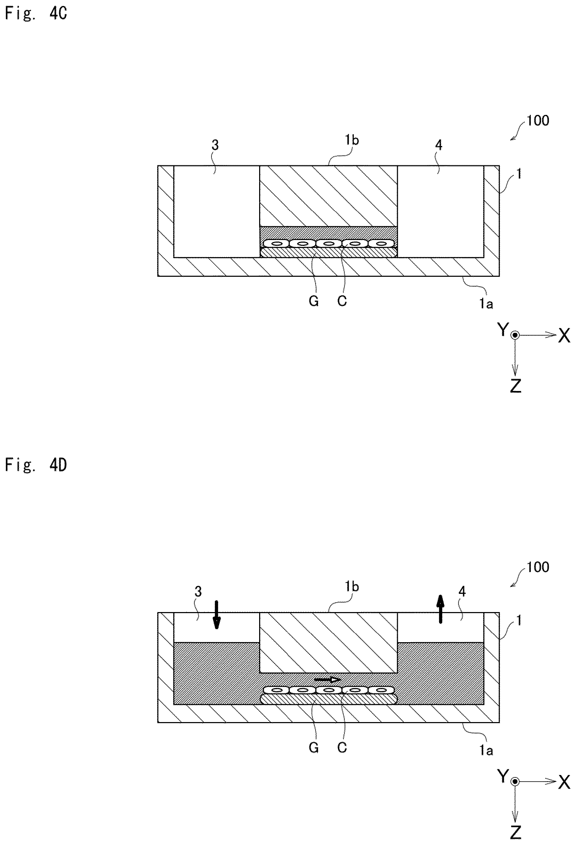

Hereinbelow, a method for using the microfluidic device 100 will be described in detail. Fig. 4A to Fig. 4D are sectional views schematically illustrating the method for using the microfluidic device 100. The method for using the microfluidic device 100 includes: a first step (shown in Fig. 4A) in which the microfluidic device 100 is disposed so that the first principal surface 1a is located on a lower side and the second principal surface 1b is located on an upper side; a second step (shown in Fig. 4B) in which a sol S is injected into a region below the side walls 22a and 22b of the second space 22 in the first flow path 2, and a third step (shown in Fig. 4C) in which cell C are seeded on a top surface of a gel G obtained by solidifying the sol S. The method for using the microfluidic device 100 may further include a fourth step (shown in Fig. 4D) in which a culture solution is injected into a region above the gel G in the first flow path 2 to culture the cells C.

The sol S herein means a solution in which colloidal particles (dispersoid) are contained in a dispersion medium, and the gel G herein refers to one obtained by solidifying the sol S into a jelly-like state. In the second step shown in Fig. 4B, a sol S is injected through the first flow path port 3 or the first flow path port 4. The amount of the sol S to be injected is equal to or less than an amount corresponding to the volume of the first space 21, preferably equal to an amount corresponding to the volume of the first space 21.

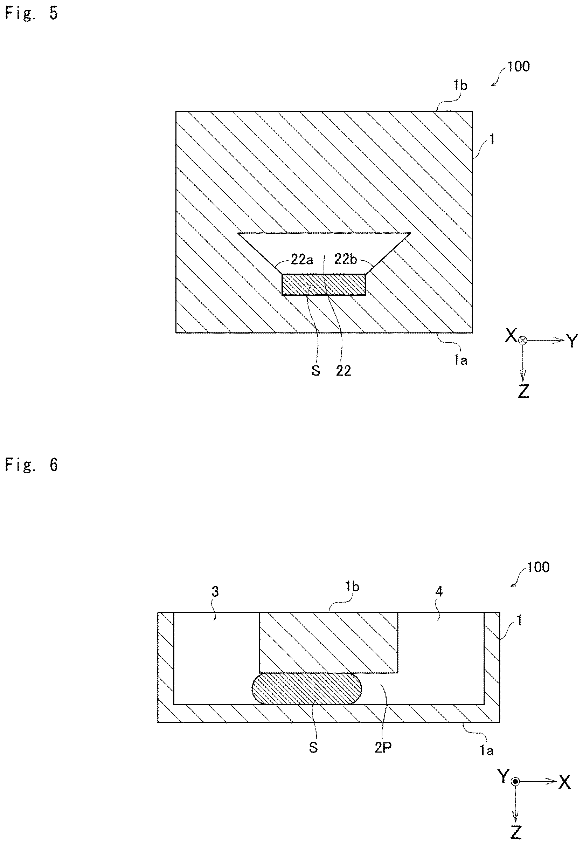

As shown in Fig. 5, the sol S injected through the first flow path port 3 or the first flow path port 4 remains in the first space 21 under the action of surface tension. Surface tension is the property of a liquid or solid to minimize its surface area as much as possible, and can quantitatively be represented as surface free energy per unit area in the unit of mJ/m².

Fig. 6 is a sectional view of a comparative example in which the flow path width of a first flow path 2P in the Y direction is constant in the Z direction. When the flow path width of the first flow path 2P is constant in the Z direction, a sol S is in contact with the ceiling of the first flow path 2P when flowing into the first flow path 2P. When the sol S is solidified in such a state, cells C cannot be seeded on the top surface of a gel, and a culture solution cannot be perfused, either.

The reason why the sol S flows into the first flow path 2P in such a manner as shown in Fig. 6 is that a liquid (here, the sol S) flows into the first flow path 2P so that the total exposed surface area of the liquid (the area of the liquid-gas interface) is minimized for energetic stability.

On the other hand, in the present invention, the total surface area of the sol S at the time when the sol S remains at the level of the interface 20 between the first space 21 and the second space 22 is smaller than that at the time when the sol S is filled beyond the interface 20, and therefore the sol S remains at the level of the interface 20 under the action of surface tension to be energetically stable.

When the sol S is solidified in such a state, the second space 22 is maintained above the gel G so that cells C can be seeded on the top surface of the gel G in the third step. It should be noted that the cells C can be seeded by injecting a culture solution containing the cells C into the second space 22. Since the side walls 22a and 22b (enlarged parts) provide surfaces inclined with respect to the first principal surface 1a, the cells C can be prevented from adhering to surfaces other than the top surface of the gel 2 when the cells C are seeded.

Further, since the second space 22 is maintained above the gel G, the cells C can be cultured by injecting a culture solution into a region above the gel G in the fourth step. The cells C after cultivation are observed through the first principal surface 1a with a microscope from the outside of the microfluidic device 100. In this regard, the microfluidic device 100 according to the present invention is excellent in observability because the top surface of the gel G is flat and the parallel to the first principal surface 1a. It should be noted that the present embodiment has been described with reference to a case where the first principal surface 1a and the top surface of the gel G are parallel to each other, but when the microfluidic device 100 has a frame surrounding the first principal surface 1a, the top surface of the gel G shall be parallel to the bottom surface of the frame.

The gel G is selected from the group consisting of collagen, gelatin, hyaluronate, hyaluronan, fibrin, alginate, agarose, chitosan, chitin, cellulose, pectin, starch, laminin, fibrinogen/thrombin, fibrillin, elastin, gum, cellulose, agar, gluten, casein, albumin, vitronectin, tenascin, entactin/nidogen, glycoprotein, glycosaminoglycan, poly(acrylic acid) and derivatives thereof, poly(ethylene oxide) and copolymers thereof, poly(vinyl alcohol), polyphosphazene, Matrigel, and combinations of two or more of them.

### <Second embodiment>

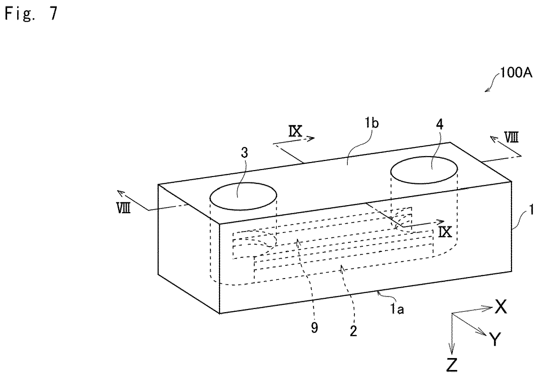

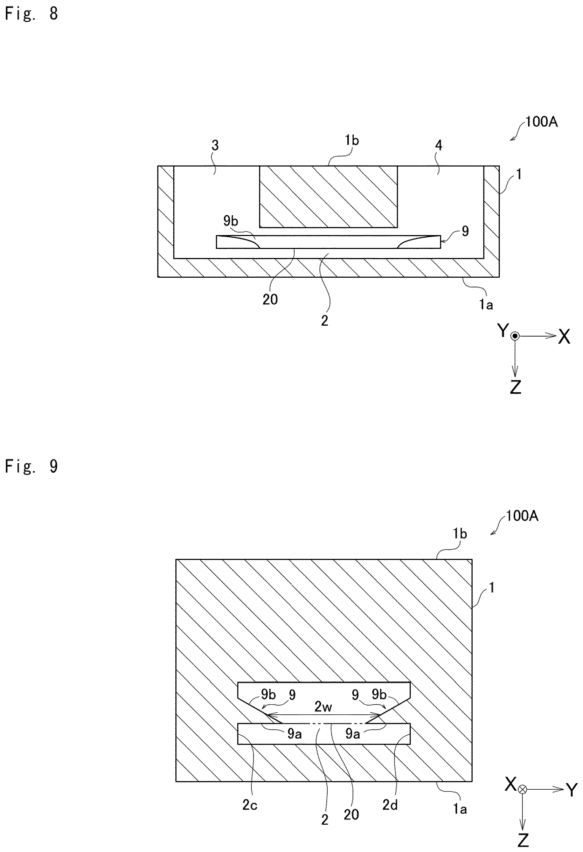

Fig. 7 is a perspective view of a microfluidic device 100A according to a second embodiment. Fig. 8 is a sectional view of the microfluidic device 100A taken along a line VIII-VIII shown in Fig. 7, and Fig. 9 is a sectional view of the microfluidic device 100A taken along a line IX-IX shown in Fig. 7.

As shown in Fig. 7 to Fig. 9, the first flow path 2 in the second embodiment includes projections 9 that project from side walls 2c and 2d in the Y direction and extend in the X direction. In the present embodiment, the projections 9 project from the central regions of both of the side walls 2c and 2d in the Z direction, respectively. Each of the projections 9 has a triangular cross-section in the YZ plane.

Each of the projections 9 has a lower surface 9a on the first principal surface 1a side (+Z direction side) and an upper surface 9b on the second principal surface 1b side (-Z direction side). The lower surface 9a is parallel to the first principal surface 1a. On the other hand, the upper surface 9b is inclined with respect to the first principal surface 1a. The upper surface 9b is inclined so as to depart from the first principal surface 1a as extending from the tip of the projection 9 toward the side wall 2c or 2d. This allows the flow path width 2w of the first flow path 2 in the Y direction to increase from the first principal surface 1a side toward the second principal surface 1b side in a region where the projections 9 are formed. That is, the upper surfaces 9b of the projections 9 in the second embodiment correspond to "enlarged parts" in the present invention. It should be noted that in a region where the projections 9 are not formed, both of the side walls 2c and 2d of the first flow path 2 in the Y direction are parallel to each other.

As described above, the microfluidic device 100A according to the second embodiment includes a plate-shaped main body 1 having a first principal surface 1a and a second principal surface 1b facing each other in the Z direction, a first flow path 2 formed inside the main body 1 and extending along the XY plane between the first principal surface 1a and the second principal surface 1b, and first flow path ports 3 and 4 extending in the Z direction and each having one end open to an end of the first flow path 2 and another end open to the second principal surface 1b. Further, the first flow path 2 has upper surfaces 9b of projections 9 (enlarged parts) so that a flow path width 2w increases from the first principal surface 1a side toward the second principal surface 1b side when the first flow path 2 is viewed in its extension direction (X direction).

Further, as described with reference to the second embodiment, the upper surfaces 9b of the projections 9 (enlarged parts) are inclined with respect to the first principal surface 1a.

Further, as described with reference to the second embodiment, the first flow path 2 has upper surfaces 9b of projections 9 (enlarged parts) on its both sides in the Y direction respectively, and a virtual surface 20 formed by connecting together the first principal surface 1a-side ends of the upper surfaces 9b is parallel to the first principal surface 1a.

Although the embodiments of the present invention have been described above with reference to the drawings, it should be understood that specific configurations are not limited to those of these embodiments. The scope of the present invention is indicated not only by the above description of the embodiments but also by the claims, and all changes which come within the meaning and range of equivalency of the claims are therefore intended to be embraced therein.

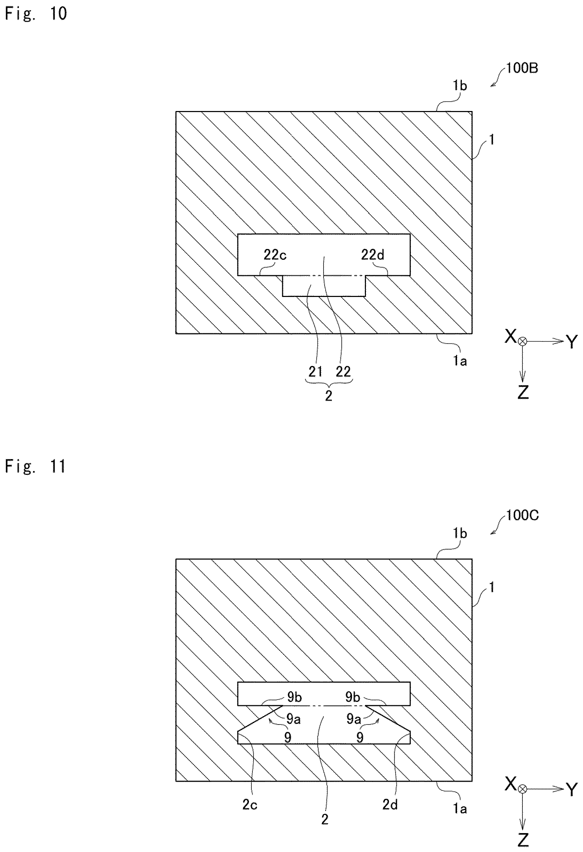

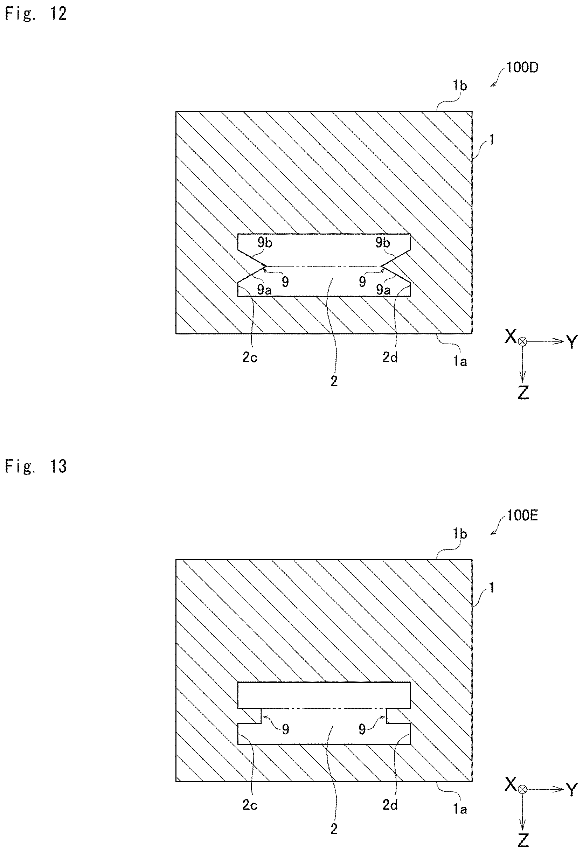

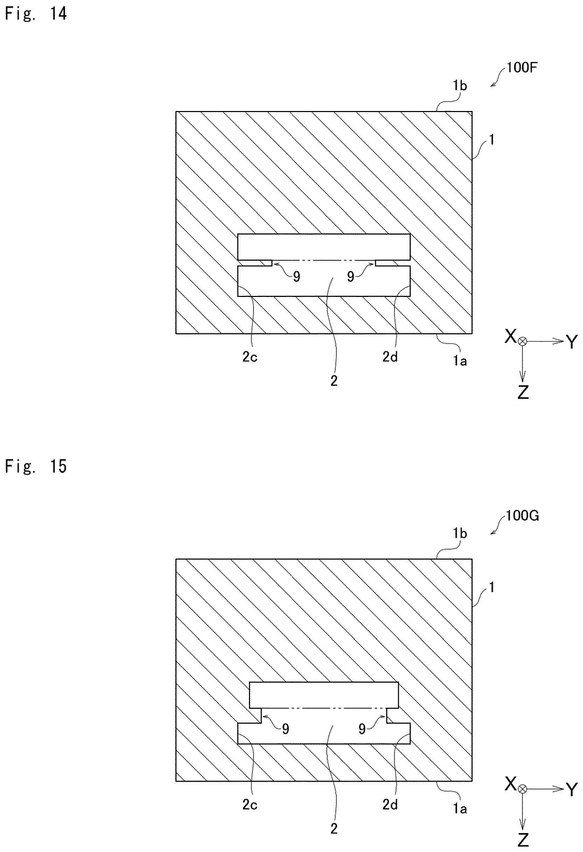

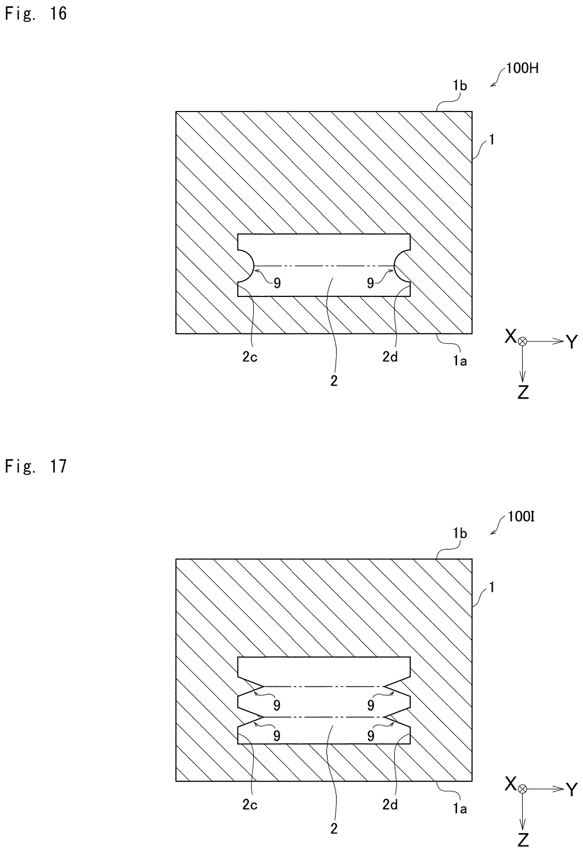

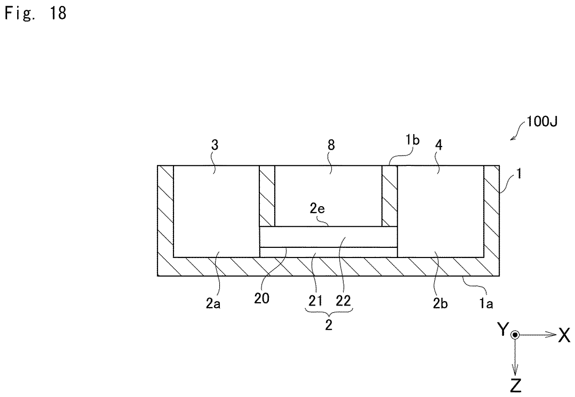

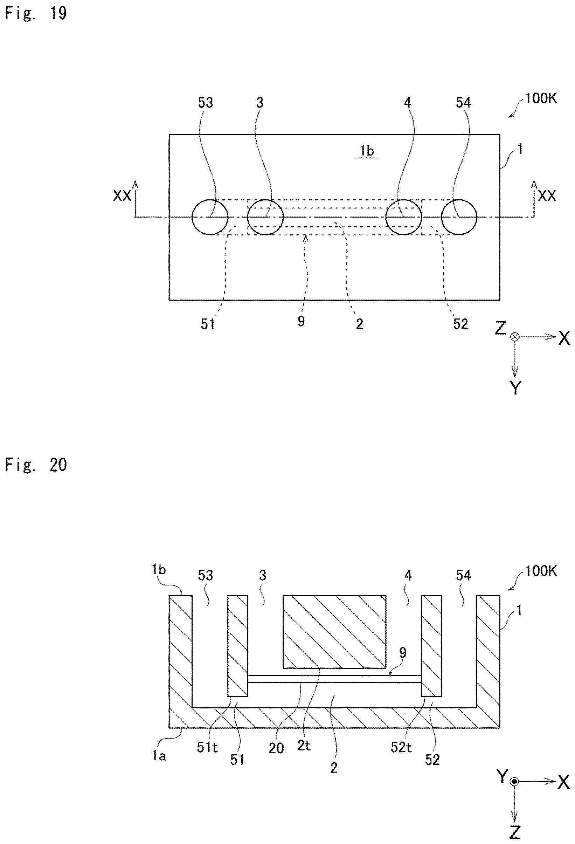

The structure adopted in each of the above embodiments can be adopted in any other embodiment. Specific configurations of parts are not limited only to those in the above-described embodiments, and various modifications can be made without departing from the spirit of the present invention.
(1) In the microfluidic device 100 according to the first embodiment, the first flow path 2 is configured to include a first space 21 and a second space 22 that is adjacent to the first space 21 on the second principal surface 1b side in the Z direction and that has a larger flow path width 2w than the first space 21 by the side walls 22a and 22b (enlarged parts). However, the configuration of the microfluidic device 100 is not limited thereto. As in the case of a microfluidic device 100B shown in Fig. 10, the first flow path 2 may include a first space 21 and a second space 22 that is adjacent to the first space 21 on the second principal surface 1b side in the Z direction and that has a larger flow path width 2w than the first space 21 by bottom walls 22c and 22d. That is, the bottom walls 22c and 22d of the second space 22 correspond to "enlarged parts" in the present invention. The second space 22 has a rectangular cross-section in the YZ plane, and the bottom walls 22c and 22d are parallel to the first principal surface 1a.
(2) The microfluidic device 100A according to the second embodiment is configured in such a manner that the upper surfaces 9b of the projections 9 are inclined with respect to the first principal surface 1a. However, the configuration of the microfluidic device 100A is not limited thereto. As in the case of a microfluidic device 100C shown in Fig. 11, the upper surfaces 9b of the projections 9 may be parallel to the first principal surface 1a. In this case, the lower surfaces 9a of the projections 9 are inclined with respect to the first principal surface 1a.
(3) The microfluidic device 100A according to the second embodiment is configured in such a manner that the lower surfaces 9a of the projections 9 are parallel to the first principal surface 1a, and the upper surfaces 9b are inclined with respect to the first principal surface 1a. However, the configuration of the microfluidic device 100A is not limited thereto. As in the case of a microfluidic device 100D shown in Fig. 12, the lower surfaces 9a of the projections 9 may also be inclined with respect to the first principal surface 1a similarly to the upper surfaces 9b.
(4) The microfluidic device 100A according to the second embodiment is configured in such a manner that the projections 9 are formed to have a triangular cross-section in the YZ plane. However, the configuration of the microfluidic device 100A is not limited thereto. As in the case of a microfluidic device 100E shown in Fig. 13, the projections 9 may be formed to have a rectangular cross-section in the YZ plane. Alternatively, as in the case of a microfluidic device 100F shown in Fig. 14, the projections 9 may be formed in a thin film shape. In this case, each of the film-shaped projections 9 has a thickness of, for example, 50 µm.
(5) The microfluidic device 100A according to the second embodiment is configured in such a manner that both of the side walls 2c and 2d of the first flow path 2 in the Y direction are parallel to each other in a region where the projections 9 are not formed. However, the configuration of the microfluidic device 100A is not limited thereto. As in the case of a microfluidic device 100G shown in Fig. 15, a region above the projections 9 and a region below the projections 9 may be different in the flow path width of the first flow path 2.
(6) Alternatively, as in the case of a microfluidic device 100H shown in Fig. 16, the projections 9 may be formed to have a semicircular cross-section in the YZ plane. In this case, 1/4 circular arcs of the semicircular projections 9 on the second principal surface 1b side correspond to "enlarged parts" in the present invention.
(7) Alternatively, as in the case of a microfluidic device 100I shown in Fig. 17, the first flow path 2 may have more than two projections 9. In this case, two or more gel layers can be formed by, for example, first forming a gel using the lower projections 9 and then forming a gel using the upper projections 9. By changing a gel composition, density, or cells to be contained from layer to layer, various tissues or organs can be simulated, such as corneas, blood vessels (aortas, venae cavae), tracheal walls, and joints.
(8) Alternatively, as in the case of a microfluidic device 100J shown in Fig. 18, a hole 8 may be provided which extends in the Z direction and has one end open to a middle part 2e of the first flow path 2 and another end open to the second principal surface 1b. Such a configuration makes it possible to culture cells seeded on the gel surface in a state where the apical side of the cells is in contact with air (air-liquid interface culture), thereby simulating an environment such as respiratory epithelial cells that interact with both liquid and air in vivo.
(9) Alternatively, as in the case of a microfluidic device 100K shown in Fig. 19 and Fig. 20, a second flow path 51 connected to the first flow path 2 and extending along the XY plane, a third flow path 52 connected to the first flow path 2 and extending along the XY plane, at least one second flow path port 53 extending in the Z direction and having one end open to the second flow path 51 and another end open to the second principal surface 1b, and at least one third flow path port 54 extending in the Z direction and having one end open to the third flow path 52 and another end open to the second principal surface 1b may be provided. In this embodiment, a second principal surface 1b-side upper wall surface 2t of the first flow path 2 is closer to the second principal surface 1b than a second principal surface 1b-side upper wall surface 51t of the second flow path 51 and a second principal surface 1b-side upper wall surface 52 t of the third flow path 52. Further, in this embodiment, the projections 9 are configured to have upper surfaces 9b corresponding to the enlarged parts as shown in Fig. 9, and the virtual surface 20 is closer to the second principal surface 1b than the second principal surface 1b-side upper wall surface 51t of the second flow path 51 and the second principal surface 1b-side upper wall surface 52t of the third flow path 52.

An example of a method for using the microfluidic device 100K shown in Fig. 19 and Fig. 20 will simply be described.

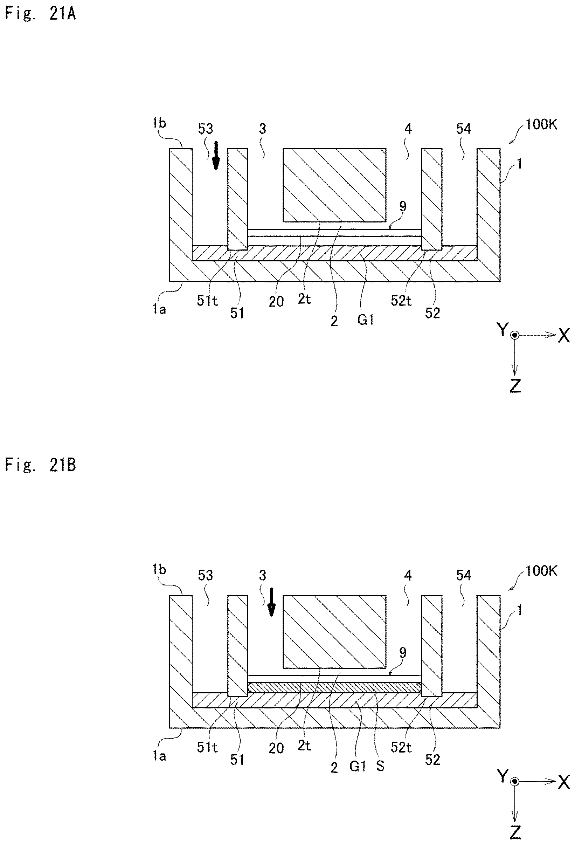

First, as shown in Fig. 21A, glycerol or thermal phase transition hydrogel G1 is injected through the second flow path port 53. At this time, the amount of the glycerol or thermal phase transition hydrogel G1 to be injected is set so that the height of the injected glycerol or thermal phase transition hydrogel G1 is higher than the upper wall surface 51t of the second flow path 51 and the upper wall surface 52t of the third flow path 52 and lower than the virtual surface 20.

Then, as shown in Fig. 21B, a sol S is injected through the first flow path port 3 so as to spread on the glycerol or thermal phase transition hydrogel G1 and is then gelatinized. At this time, the sol S is injected into a region below the virtual surface 20 in the first flow path 2.

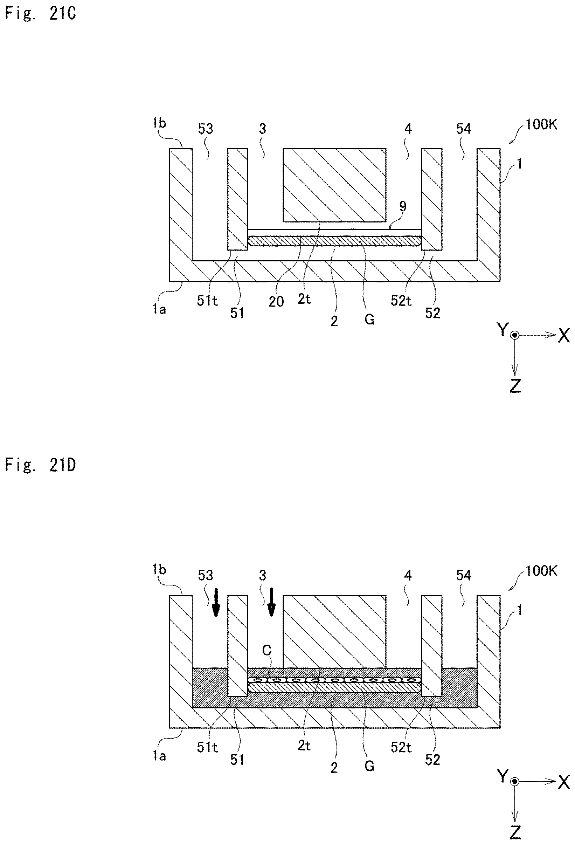

Then, as shown in Fig. 21C, the glycerol or thermal phase transition hydrogel G1 is removed through the second flow path port 53 or the third flow path port 54. As a result, a gel G remains, and spaces are created above and below the gel G in the first flow path 2.

Then, as shown in Fig. 21 D, cells C are seeded on the upper side of the gel G.

### DESCRIPTION OF REFERENCE SIGNS

- 100: Microfluidic device
- 100A: Microfluidic device
- 100B: Microfluidic device
- 100C: Microfluidic device
- 100D: Microfluidic device
- 100E: Microfluidic device
- 100F: Microfluidic device
- 100G: Microfluidic device
- 100H: Microfluidic device
- 100I: Microfluidic device
- 100J: Microfluidic device
- 100K: Microfluidic device
- 1: Main body
- 1a: First principal surface
- 1b: Second principal surface
- 2: First flow path
- 2a: One end
- 2b: Another end
- 2c: Side wall
- 2d: Side wall
- 2e: Middle part
- 2t: Upper wall surface
- 2w: Flow path width
- 3: First flow path port
- 4: First flow path port
- 8: Hole
- 9: Projection
- 9a: Lower surface of projection
- 9b: Upper surface of projection
- 20: Virtual surface (Interface)
- 21: First space
- 22: Second space
- 22a: Side wall of second space
- 22b: Side wall of second space
- 22c: Bottom wall of second space
- 22d: Bottom wall of second space
- 51: Second flow path
- 51t: Upper wall surface of second flow path
- 52: Third flow path
- 52t: Upper wall surface of third flow path
- 53: Second flow path port
- 54: Third flow path port
- C: Cell
- G: Gel
- G1: Glycerol or thermal phase transition hydrogel
- S: Sol

## Claims

1. A microfluidic device comprising: a plate-shaped main body having a first principal surface and a second principal surface facing each other in a first direction; a first flow path formed inside the main body and extending along a plane between the first principal surface and the second principal surface; and a plurality of first flow path ports extending in the first direction and each having one end open to an end of the first flow path and another end open to the second principal surface, wherein
the first flow path has an enlarged part so that a flow path width increases from a first principal surface side toward a second principal surface side when the first flow path is viewed in its extension direction.

2. The microfluidic device according to claim 1, wherein the first flow path includes a first space and a second space that is adjacent to the first space on the second principal surface side and that has a larger flow path width than the first space by the enlarged part.

3. The microfluidic device according to claim 1, wherein
the first flow path includes a projection that projects from a side wall and extends in the extension direction, and
the enlarged part corresponds to a second principal surface-side surface of the projection.

4. The microfluidic device according to claim 1, wherein the enlarged part is a surface parallel to the first principal surface.

5. The microfluidic device according to claim 1, wherein the enlarged part is a surface inclined with respect to the first principal surface.

6. The microfluidic device according to claim 1, wherein
the first flow path has the enlarged parts on its both sides in a width direction respectively, and
a virtual surface formed by connecting together first principal surface-side ends of the enlarged parts is parallel to the first principal surface or a bottom surface of a frame surrounding a periphery of the first principal surface.

7. The microfluidic device according to claim 1, comprising a hole extending in the first direction and having one end open to a middle part of the first flow path and another end open to the second principal surface.

8. The microfluidic device according to claim 1, comprising:
a second flow path connected to the first flow path and extending along the plane;
a third flow path connected to the first flow path and extending along the plane;
at least one second flow path port extending in the first direction and having one end open to the second flow path and another end open to the second principal surface; and
at least one third flow path port extending in the first direction and having one end open to the third flow path and another end open to the second principal surface.

9. A method for using the microfluidic device according to claim 1, the method comprising:
a first step in which the microfluidic device is disposed so that the first principal surface is located on a lower side and the second principal surface is located on an upper side;
a second step in which a sol is injected into a region below the enlarged part in the first flow path, and
a third step in which cells are seeded on a top surface of a gel obtained by solidifying the sol.

10. The method for using the microfluidic device according to claim 9, comprising a fourth step in which a culture solution is injected into a region above the gel in the first flow path to culture the cells.

11. The method for using the microfluidic device according to claim 9, wherein the gel is selected from the group consisting of collagen, gelatin, hyaluronate, hyaluronan, fibrin, alginate, agarose, chitosan, chitin, cellulose, pectin, starch, laminin, fibrinogen/thrombin, fibrillin, elastin, gum, cellulose, agar, gluten, casein, albumin, vitronectin, tenascin, entactin/nidogen, glycoprotein, glycosaminoglycan, poly(acrylic acid) and derivatives thereof, polyethylene oxide) and copolymers thereof, poly(vinyl alcohol), polyphosphazene, Matrigel, and combinations of two or more of them.
